# FASCICULE DE BREVET EUROPEEN

(11) **EP 2 300 039 B1**
(45) Date de publication et mention de la délivrance du brevet: **24.08.2016**
(21) Numéro de dépôt: 09757615.1
(22) Date de dépôt: 05.06.2009
(51) Int. Cl.: C07K 14/47, A61K 38/01, A61K 38/17, A23L 33/18, A61P 25/22, A61P 25/20, A61P 25/08, A61K 38/08, C12N 15/12

(54) **COMPOSITIONS ANXIOLYTIQUES COMPRENANT DES PEPTIDES DERIVES DE LA CASEINE ALPHAs¹**
ANXIOLYTISCHE ZUSAMMENSETZUNG MIT PEPTIDEN VON ALPHAS1-CASEIN
ANXIOLYTIC COMPOSITION CONTAINING ALPHAs¹-CASEIN -DERIVED PEPTIDES

(30) Priorité: 06.06.2008 FR 0853757
(43) Date de publication de la demande: 30.03.2011
(73) Titulaire: Université de Lorraine, 54052 Nancy Cedex (FR)
(72) Inventeur: BALANDRAS, Frédérique, F-54000 Nancy (FR); GAILLARD, Jean-Luc, F-14530 Luc sur Mer (FR); LAURENT, François, 54000 NANCY (FR); LE ROUX, Yves, F-54500 Vandoeuvre-les-Nancy (FR); MICLO, Laurent, F-54600 Villers-les-Nancy (FR)
(74) Mandataire: Schmidt, Martin Peter
(86) Numéro de dépôt international: PCT/EP2009/056933
(87) Numéro de publication internationale: WO 2009/147234

(56) Documents cités:
- EP-A- 0 714 910
- EP-A1- 1 188 767
- LOUKAS S.: "Opioid activities and structures of alpha-casein-derived exorphins." BIOCHEMISTRY, vol. 22, 1983, pages 4567-4573, XP009111701 cité dans la demande
- GUESDON ET AL: "A tryptic hydrolysate from bovine milk alphaS1-casein improves sleep in rats subjected to chronic mild stress" PEPTIDES, ELSEVIER, AMSTERDAM, vol. 27, no. 6, 1 juin 2006 (2006-06-01), pages 1476-1482, XP005458217 ISSN: 0196-9781

### SEQUENCE LISTING

<110> Institut National Polytechnique de Lorraine Université Henri-Poincaré Nancy 1
<120> Compositions anxiolytiques comprenant des peptides dérivés de la caséine alpha S1
<130> 354723 D26668
<150> FR 0853757
   <151> 2008-06-06
<160> 2
<170> PatentIn version 3.3
<210> 1
   <211> 5
   <212> PRT
   <213> Bos taurus
<400> 1
   Tyr Leu Gly Tyr Leu
   1               5
<210> 2
   <211> 6
   <212> PRT
   <213> Bos taurus
<400> 2
   Tyr Leu Gly Tyr Leu Glu
   1               5

## Description

La présente invention concerne des compositions pharmaceutiques et des compositions alimentaires comprenant des peptides dérivés de la caséine αₛ₁ ayant une activité anxiolytique.

La caséine donne, par diverses techniques de fractionnement, les principales fractions qui sont dénommées respectivement : caséine κ, caséine β, caséine αₛ₁ et caséine αₛ₂. Les séquences en acides aminés de ces caséines sont bien connues, en particulier celle de la caséine αₛ₁ a été déterminée par MERCIER *et al.* (1) et NAGAO *et al.* (2).

Il a été montré que certains fragments peptidiques de ces différentes caséines ont des activités biologiques diverses et notamment des activités opiacées ou anti-opiacées et des activités inhibitrices de l'enzyme de conversion de l'angiotensine I. Ainsi, les peptides 90-96 et 90-95 de la caséine αₛ₁ ont une activité opiacée démontrée *in vitro* [ZIOUDROU *et al.* (3) et LOUKAS *et al.* (4)]. Le résidu d'arginine en position 90 semble important pour cette activité opiacée. Les peptides 91-95 et 91-96 dans lesquels le résidu d'arginine est supprimé sont pratiquement inactifs et ils ont donc une activité opiacée très inférieure au peptide 90-96. Les peptides 23-34 et 194-199, quant à eux, sont des inhibiteurs de l'enzyme de conversion de l'angiotensine I [MARUYAMA et SUZUKI (5) et MARUYAMA *et al.* (6)].

Un peptide présentant une activité originale de type anxiolytique a été mis en évidence dans un hydrolysat trypsique de caséine αₛ₁ [EP 0714910 (7)] Il correspond au fragment 91-100 et a été nommé α-casozépine [MICLO *et al.* (8)]. Guesdon *et al.* (18) ont également montré que ce même hydrolysat trypsique de la caséine comprenant le peptide 91-100 améliore le sommeil chez des rats soumis à un stress chronique. La structure de ce décapeptide a été étudié par ¹H-RMN bidimensionnelle. La séquence comprise entre les résidus de glycine 93 et de leucine 99 adopte, en milieu micellaire, une structure en hélice 3₁₀ initiée et terminée par un tour d'hélice α. Les chaînes latérales des résidus hydrophobes se situent sur la même face de l'hélice alors que les chaînes latérales des résidus hydrophiles se situent sur l'autre face, ce qui confère un caractère amphiphile au peptide et peut lui permettre des interactions avec les membranes. Les interactions ioniques entre le groupement guanidinium du résidu d'arginine 100 et les groupements carboxyliques des résidus d'acide glutamique 96 et d'arginine 100 montrent le rôle capital du résidu d'arginine carboxy-terminal dans la stabilisation de la structure hélicoïdale. Dans une telle structure, les noyaux aromatiques des deux résidus de tyrosine en position 91 et 94 sont orientés de telle façon que la distance entre leur centre (0,56 nm en moyenne) est comparable à la distance observée entre les centres des noyaux aromatiques du nitrazépam, benzodiazépine connue pour ses propriétés anxiolytiques [LECOUVEY *et al.* (9)]. La substitution du résidu d'arginine 100 par un résidu d'alanine diminue fortement l'hélicité du décapeptide et il en résulte une diminution de l'affinité de ce peptide pour le site benzodiazépine du récepteur GABA_{A} d'un facteur 300.000 [Thèse Céline Frochot, (10)]. Ce décapeptide, après absorption orale, peut subir des attaques protéolytiques par les enzymes du tractus digestif ce qui peut diminuer sa biodisponibilité et par conséquent l'empêcher d'atteindre sa cible biologique. Or il est bien connu pour les petits peptides que l'absorption entérocytaire est plus efficace que celle de fragments plus importants et que leur résistance vis-à-vis des protéases digestives est accrue. Ainsi, parmi les fragments générés au cours de la digestion du décapeptide, certains pourraient être plus absorbables et plus résistants, mais au vu des données structurales issues du décapeptide, sans pouvoir conserver la moindre activité de type anxiolytique.

La demande de brevet pendante PCT/EP2007/063863 montre de manière surprenante que les peptides 91-97, 91-98, 91-99 dérivés du décapeptide et ne contenant pas le résidu-clé d'arginine 100 montrent dans des tests comportementaux *in vivo* chez le rat des propriétés anxiolytiques. Au vu de leur petite taille, ces peptides sont plus facilement absorbables et moins facilement dégradables.

La présente demande concerne maintenant des compositions pharmaceutiques et des produits alimentaires ayant des propriétés anxiolytiques comprenant les peptides 91-95 et 91-96 dérivés de la caséine αₛ1. Les propriétés anxiolytiques de ces peptides ont pu être démontrées dans des tests comportementaux *in vivo* chez le rat.

### Listage des séquences

SEQ ID NO. 1 : Pentapeptide correspondant aux positions 91-95 de la caséine αₛ1
SEQ ID NO. 2 : Hexapeptide correspondant aux positions 91-96 de la caséine αₛ1

### Description de l'invention

L'invention a pour objet des compositions pharmaceutiques comprenant à titre de principe actif une quantité efficace d'un peptide selon la SEQ ID No. 1 en combinaison avec un véhicule pharmaceutique approprié.

Les compositions pharmaceutiques selon l'invention ont une activité de type benzodiazépine et conviennent plus particulièrement pour le traitement de l'anxiété, des troubles du sommeil et de l'épilepsie.

Dans un mode de réalisation particulier, l'invention concerne des compositions pharmaceutiques comprenant un hydrolysat ou une fraction d'un hydrolysat de la caséine ou de la caséine αₛ1, contenant une quantité efficace d'un peptide selon la SEQ ID No. 1

L'invention se rapporte aussi à un produit alimentaire pour les personnes sujettes notamment à l'anxiété, aux troubles du sommeil et/ou à l'épilepsie comprenant une quantité efficace d'au moins un peptide selon la SEQ ID No. 1.

Dans un mode de réalisation avantageux de l'invention, le produit alimentaire comprend un hydrolysat ou une fraction d'un hydrolysat de la caséine ou de la caséine αₛ1, contenant une quantité efficace d'un peptide selon la SEQ ID No. 1.

L'invention a aussi pour objet des polynucléotides isolés codant pour un peptide selon la SEQ ID No. 1.

L'invention se rapporte également à des vecteurs d'expression comprenant un polynucléotide selon l'invention.

Un autre objet de l'invention est un organisme hôte, à l'exclusion de l'homme, transformé avec un polynucléotide selon l'invention et/ou avec un vecteur d'expression selon l'invention.

L'invention a également pour objet un organisme hôte transformé, à l'exclusion de l'homme, exprimant un peptide selon la SEQ ID No. 1.

L'invention concerne aussi l'utilisation d'un peptide selon la SEQ ID No. 1, d'un peptide selon la SEQ ID No. 2, d'un polynucléotide selon l'invention, d'un vecteur d'expression selon l'invention et/ou d'un organisme hôte selon l'invention pour la fabrication d'un médicament pour le traitement de l'anxiété, des troubles du sommeil et de l'épilepsie.

De préférence, l'invention concerne l'utilisation d'un hydrolysat de la caséine ou de la caséine αₛ1 et/ou d'une fraction d'un hydrolysat de la caséine ou de la caséine αₛ1 contenant une quantité efficace d'un peptide selon la SEQ ID No. 1 et/ou d'un peptide selon la SEQ ID No. 2 pour la fabrication d'un médicament pour le traitement de l'anxiété, des troubles du sommeil et de l'épilepsie.

Préférentiellement le médicament a une activité de type benzodiazépine.

Un autre objet de l'invention est l'utilisation d'un peptide selon la SEQ ID No. 1, d'un peptide selon la SEQ ID No. 2, d'un polynucléotide selon l'invention, d'un vecteur d'expression selon l'invention et/ou d'un organisme hôte selon l'invention pour la fabrication d'un produit alimentaire pour les personnes sujettes notamment à l'anxiété, aux troubles du sommeil et/ou à l'épilepsie.

De manière préférée, l'invention a pour objet l'utilisation d'un hydrolysat de la caséine ou de la caséine αₛ1 et/ou d'une fraction d'un hydrolysat de la caséine ou de la caséine αₛ1 contenant une quantité efficace d'un peptide selon la SEQ ID No. 1 et/ou d'un peptide selon la SEQ ID No. 2 pour la fabrication d'un produit alimentaire pour les personnes sujettes notamment à l'anaciété, aux troubles du sommeil et/ou à l'épilepsie.

Ainsi la présente invention a pour objet des compositions pharmaceutiques et des produits alimentaires comprenant des peptides dérivés de la caséine αₛ1 dont la séquence est représentée aux SEQ ID NOs. 1. L'invention concerne donc des compositions pharmaceutiques et des produits alimentaires contenant des peptides dont la séquence en acides aminés est choisie parmi : Tyr-Leu-Gly-Tyr-Leu.

L'invention se rapporte également à des compositions pharmaceutiques et à des produits alimentaires comprenant des peptides modifiés ayant la SEQ ID NO. 1. et conservant leurs propriétés anxiolytiques. L'invention se rapporte également à des compositions pharmaceutiques et à des produits alimentaires comprenant des protéines de fusion ou des protéines recombinantes comprenant le peptide selon la SEQ ID No. 1

Dans un deuxième aspect, l'invention se rapporte à des polynucléotides codant pour les peptides selon la SEQ ID No. 1. En raison de la dégénérescence du code génétique, différents polynucléotides peuvent coder pour un même peptide. Selon la présente invention, on entend par "polynucléotide " une chaine nucléotidique simple brin ou son complémentaire pouvant être de type ADN ou ARN, ou une chaine nucléotidique double brin pouvant être de type ADNc (complémentaire) ou génomique. De préférence, les polynucléotides de l'invention sont de type ADN, notamment d'ADN double brin. Le terme "polynucléotide" désigne également les polynucléotides modifiés. De préférence, les polynucléotides de la présente invention peuvent être préparés par les techniques classiques de biologie moléculaire telles que décrites par Sambrook et al. (Molecular Cloning : A Labratory Manual, 1989) ou par synthèse chimique.

Un autre objet de l'invention est un organisme hôte exprimant un peptide selon la SEQ ID NOs. 1. Les peptides selon la SEQ ID No. 1 et la SEQ ID No. 2 peuvent être exprimés et produits dans différents organismes hôtes selon des techniques bien connues de l'homme du métier. Typiquement, l'organisme hôte est transformé avec une cassette d'expression comprenant un polynucléotide codant pour un peptide selon la SEQ ID No. 1 ou la SEQ ID No. 2. Ce polynucléotide peut être intégré dans le génome de l'organisme hôte ou se répliquer de manière stable dans l'organisme hôte. Par organisme hôte, on entend en particulier selon l'invention tout organisme mono ou pluricellulaire, inférieur ou supérieur, en particulier choisi parmi les bactéries, les levures, les champignons et les mammifères. Par organisme hôte, on entend un organisme non humain. Les peptides selon la SEQ ID No. 1 ou la SEQ ID No. 2 peuvent donc être produits puis isolés ou purifiés à partir d'organismes hôtes transformés les exprimant.

De préférence, les peptides selon la SEQ ID No. 1 ou la SEQ ID No. 2 sont obtenus à partir de la caséine du lait et plus préférentiellement à partir de la caséine αₛ1. La caséine utilisée est de préférence la caséine du lait de bovins et plus préférentiellement de vaches laitières (*Bos taurus*). La séquence de la caséine αₛ1 est référencée dans la base de données Swiss Prot sous le numéro P02662. Après fractionnement des protéines du lait, la caséine est « digérée » ou hydrolysée avec des enzymes appropriées pour obtenir les peptides selon l'invention. Dans un premier mode de réalisation, la caséine du lait est directement hydrolysée avec des enzymes pour obtenir les peptides souhaités. Dans ce mode de réalisation, il peut être nécessaire de purifier partiellement ou totalement les peptides selon la SEQ ID No. 1 ou la SEQ ID No. 2 après l'hydrolyse. Dans un deuxième mode de réalisation selon l'invention, l'hydrolyse enzymatique s'effectue directement sur de la caséine αₛ1. Dans ce mode de réalisation, l'hydrolysat obtenu sera déjà enrichi en peptides selon la SEQ ID No. 1 et des étapes de purification supplémentaires ne sont souvent pas nécessaires. L'homme du métier choisira les enzymes appropriées pour obtenir les peptides souhaités. Ces techniques sont bien connues de l'homme du métier et décrites dans la littérature. La demande decrit également pour objet un hydrolysat de la caséine comprenant un peptide selon la SEQ ID No. 1 ou la SEQ ID No. 2. De préférence, il s'agit d'un hydrolysat de la caséine αₛ1.

Les compositions pharmaceutiques et les produits alimentaires selon la présente invention ont une activité de type benzodiazépine et notamment un effet anxiolytique.

L'invention concerne des compositions pharmaceutiques ayant une activité de type benzodiazépine notamment pour le traitement de l'anxiété, des troubles du sommeil, de l'épilepsie.

L'invention se rapporte donc aussi à des compositions pharmaceutiques contenant à titre de principe actif une quantité efficace d'un peptide selon la SEQ ID No. 1 en combinaison avec un véhicule pharmaceutique approprié.

Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Les compositions selon l'invention peuvent être employées en thérapie seules, ou en combinaison avec au moins un autre agent actif. Ces autres agents actifs sont en particulier choisis parmi les actifs appropriés pour le traitement de l'anxiété, des troubles du sommeil et de l'épilepsie. Il peut s'agir d'adjuvants permettant d'améliorer l'activité des composés selon l'invention, ou encore d'autres actifs connus pour leur emploi dans le traitement des dites affections. De tels agents actifs sont bien connus de l'homme du métier, disponibles dans le commerce ou encore décrits dans des ouvrages de référence comme Le Dictionnaire Vidal, édité avec mises à jours chaque année.

La présente invention concerne donc également une composition pharmaceutique comprenant un peptide selon la SEQ :ID No. 1 ainsi qu'un autre agent actif comme produit de combinaison pour une utilisation simultanée, séparée ou étalée dans le temps en thérapie, et en particulier dans le traitement de l'anxiété et des troubles du sommeil. Ces autres agents actifs sont en particulier choisis parmi les actifs appropriés pour le traitement de l'anxiété comme par exemple les composés de la classe des benzodiazépines ou les inhibiteurs de la recapture de la sérotonine.

L'invention concerne aussi l'utilisation d'un peptide selon la SEQ ID No. 1, d'un peptide selon la SEQ ID No. 2, d'un polynucléotide selon l'invention, d'un vecteur d'expression selon l'invention et/ou d'un organisme hôte selon l'invention pour la fabrication d'un médicament pour le traitement de l'anxiété, des troubles du sommeil et de l'épilepsie.

De préférence, l'invention concerne l'utilisation d'un hydrolysat de la caséine ou de la caséine αₛ1 et/ou d'une fraction d'un hydrolysat de la caséine ou de la caséine αₛ1, contenant une quantité efficace d'un peptide selon la SEQ ID No. 1 et/ou d'un peptide selon la SEQ ID No. 2 pour la fabrication d'un médicament pour le traitement de l'anxiété, des troubles du sommeil et de l'épilepsie.

L'invention concerne aussi des méthodes de traitement thérapeutique de l'anxiété, des troubles du sommeil et de l'épilepsie comprenant l'administration à un individu d'une quantité efficace d'un peptide selon la SEQ ID No. 1, d'un peptide selon la SEQ ID No. 2, d'un polynucléotide selon l'invention, d'un vecteur d'expression selon l'invention et/ou d'un organisme hôte selon l'invention.

La demande concerne aussi des méthodes de traitement thérapeutique de l'anxiété, des troubles du sommeil et de l'épilepsie comprenant l'administration à un individu d'un hydrolysat de la caséine ou de la caséine αₛ₁ et/ou d'une fraction d'un hydrolysat de la caséine ou de la caséine αₛ1, contenant une quantité efficace d'un peptide selon la SEQ ID No. 1 et/ou d'un peptide selon la SEQ ID No. 2.

La demande décrit est l'utilisation d'un peptide selon la SEQ ID No. 1, d'un peptide selon la SEQ ID No. 2, d'un polynucléotide selon l'invention, d'un vecteur d'expression selon l'invention et/ou d'un organisme hôte selon l'invention pour la fabrication d'un produit alimentaire pour les personnes sujettes notamment à l'anxiété, aux troubles du sommeil et/ou à l'épilepsie.

La demande décrit d'un hydrolysat de la caséine ou de la caséine αₛ1 et/ou d'une fraction d'un hydrolysat de la caséine ou de la caséine αₛ1 contenant une quantité efficace d'un peptide selon la SEQ ID No. 1 et/ou d'un peptide selon la SEQ ID No. 2 pour la fabrication d'un produit alimentaire pour les personnes sujettes notamment à l'anxiété, aux troubles du sommeil et/ou à l'épilepsie.

Par produit alimentaire, on entend tout ce qui est propre à servir d'aliment. Les peptides de la SEQ ID No. 1 peuvent être utilisés comme principe actif soit dans des produits alimentaires en combinaison avec des supports alimentaires de nature protéique, glucidique ou lipidique, soit dans des produits alimentaires destinés à une alimentation particulière. Les produits alimentaires de la présente invention peuvent aussi se présenter sous forme de compléments alimentaires. Ces compléments alimentaires conviennent pour supplémenter l'alimentation des personnes sujettes notamment à l'anxiété, aux troubles du sommeil et à l'épilepsie.

L'invention concerne également des procédés d'obtention des peptides de la SEQ ID No. 1 et de la SEQ ID No. 2. La caséine entière est obtenue à partir du lait par précipitation acide et neutralisation à l'aide d'un alcali selon des procédés bien connus. Par exemple, on peut utiliser la méthode de NITSCHMANN et LEHMANN (11). La caséine ou la caséine αₛ1, utilisées comme produit de départ pour l'obtention des peptides selon la SEQ ID No. 1 et la SEQ ID No. 2, peuvent être obtenues par les procédés classiques bien connus de l'homme du métier à partir du lait, de caséines entières, de caséinates et de concentrés de protéines totales du lait, obtenus par exemple selon les procédés décrits par THOMSON (12) et MAUBOIS (13). Par exemple, on peut préparer la caséine αₛ₁ en mettant en oeuvre la méthode décrite par SANOGO *et al.* (14). Cette méthode est une méthode de fractionnement sur DEAE-cellulose utilisant un gradient discontinu de chlorure de calcium comme éluant. Elle présente l'avantage de séparer rapidement l'ensemble des caséines. Elle peut être avantageusement mise en oeuvre avec, comme support échangeur d'anions, la DEAE-cellulose DE 52 [commercialisée par WHATMAN *Ltd,* Springfeld, Grande-Bretagne] qui est une résine pré-conditionnée ne nécessitant aucun pré-cycle acido-basique avant sa première utilisation. Les peptides peuvent ensuite être obtenus par hydrolyse de la caséine avec des enzymes appropriées. Les peptides peuvent ensuite être concentrés ou isolés par chromatographie liquide haute performance (CLHP) en phase inverse, par chromatographie liquide haute performance d'échange d'anions ou par chromatographie de gel filtration de seuil 1.000 Da ou par centrifugation sur membrane et autres techniques de séparation sur membrane (microfiltration, ultrafiltration, etc.).

Un autre objet de la présente invention est donc un procédé de préparation de peptides ayant une activité de type benzodiazépine et notamment une activité anxiolytique caractérisé en ce qu'il comprend les étapes suivantes :
- hydrolyse enzymatique de la caséine à l'aide de la trypsine et ensuite de la pepsine ou d'un mélange d'enzymes pancréatique;
- isolement du peptides-ayant la SEQ ID Nos. 1.

Par isolement, on entend la purification partielle ou totale des peptides ou simplement l'enrichissement de l'hydrolysat obtenu en peptides selon la SEQ ID No.1 et la SEQ ID No. 2. Cet enrichissement peut s'effectuer par exemple par fractionnement de l'hydrolysat obtenu. Alternativement, l'hydrolysat obtenu à partir de caséine αₛ₁ contenant le peptide selon la SEQ ID No. 1 peut être utilisé directement pour l'obtention des compositions pharmaceutiques et des produits alimentaires selon l'invention.

Les peptides peuvent aussi être obtenus par synthèse peptidique selon les procédés bien connus de l'homme de l'art, tels que ceux décrits par exemple par MERIFFIELD (15).

L'invention va être maintenant décrite plus en détail par les exemples ci-après non limitatifs.

### Figures

- Figure 1 :: Test du labyrinthe en croix surélevé
- Figure 2 :: Test de la boîte claire/obscure

### Exemples

### 1. Modalités d'obtention du pentapeptide

La caséine entière est obtenue à partir du lait par précipitation acide et neutralisation à l'aide d'un alcali selon des procédés bien connus.

Le pentapeptide ayant la séquence en acides aminés ci-après : Tyr-Leu-Gly-Tyr-Leu de masse moléculaire de 627 Da, correspond au peptide 91-95 de la caséine α_{s1.} Il peut être obtenu à partir de la caséine αₛ₁ par hydrolyse enzymatique, en particulier à l'aide de la trypsine et ensuite de la pepsine. L'hydrolyse par la trypsine de la caséine αₛ₁ libère des fragments peptidiques divers. L'ensemble des fragments trypsiques subit une hydrolyse par la pepsine A (EC 3.4.23.1) (issu de muqueuse gastrique porcine, avec une activité de 3.200-4.500 unités/mg de protéine), dans un rapport E/S=1/200 à un pH acide, dans un tampon et à une température optimum pour l'activité de l'enzyme pendant 240 minutes. Cette dernière hydrolyse libère de nouveaux fragments dont le pentapeptide 91-95 de la caséine αₛ₁. Ce pentapeptide peut être également généré à partir des fragments trypsiques de la caséine αₛ₁ par une hydrolyse à l'aide de la Corolase PP®, un mélange d'enzymes pancréatiques. L'hydrolyse est réalisée pendant 240 min dans un rapport E/S=1/100 à un pH, une température et dans un tampon optimum pour ce mélange enzymatique. Le pentapeptide peut être ensuite concentré ou isolé par chromatographie liquide haute performance (CLHP) en phase inverse, par chromatographie liquide haute performance d'échange d'anions ou par chromatographie de gel filtration ou par centrifugation sur membrane et autres techniques de séparation sur membrane (micro filtration, ultrafiltration, etc.).

### 2. Etudes pharmaco-comportementales chez le rat Wistar

### 2.1. Labyrinthe en croix surélevé

### 2.1.1. Principe

Le test du labyrinthe en croix surélevé permet de mesurer la densité du comportement exploratoire dans une situation nouvelle aversive [PELLOW *et al.,* 1985 (16)].

L'expérience exploite le conflit, chez les rongeurs, entre la peur des espaces ouverts et le désir d'explorer un nouvel environnement. Le rat, placé au centre du dispositif, est contraint d'explorer son environnement. Les bras ouverts constituent un environnement anxiogène, alors que ce n'est pas le cas des bras fermés. Le nombre d'entrées dans l'ensemble des bras, celui concernant les bras ouverts, les bras fermés, le temps passé dans les bras ouverts, la latence d'entrée dans le premier bras ouvert constituent des paramètres reflétant le degré d'anxiété de l'animal.

### 2.1.2. Appareillage

Le labyrinthe en croix surélevé, construit en bois, est constitué de quatre branches à 90° les unes des autres. Les deux branches ouvertes (45 x 10 cm) et les deux branches fermées (45 x 10 cm ; entourées de plaques de bois sur une hauteur de 40 cm) sont disposées en vis-à-vis. Le carré central mesure 10 x 10 cm. Les branches ouvertes sont éclairées par une lumière blanche d'une intensité de 500 lux. Le labyrinthe en croix surélevé se situe à 50 cm du sol.

### 2.1.3. Protocole

Les observations ont lieu entre 9 h et 11 h du matin pour minimiser l'influence du rythme circadien. Avant chaque passage dans le labyrinthe en croix surélevé, l'animal est placé pendant 10 min dans un openfield afin de stimuler son comportement exploratoire. Il est ensuite placé dans le carré central du labyrinthe en croix surélevé et observé pendant 5 min. Le labyrinthe en croix est lavé avec de l'éthanol à 95% (v/v) entre deux passages afin d'éliminer les odeurs.

### 2.1.4. Traitements

L'étude a portée sur 45 rats répartis en trois groupes :
- un groupe témoin (15 rats) ayant reçu 2 mL/kg d'une solution contenant 1% (v/v) de glycérol et 0,2% (v/v) de méthylcellulose (véhicule),
- un groupe traité avec du diazépam (Valium^{®}, Roche, Neuilly-sur-Seine, France) à la dose de 1 mg/kg mis en suspension dans le véhicule (15 rats) servant de contrôle positif,
- un groupe traité avec le fragment 91-95 de la caséine αₛ₁, préalablement dissous dans le véhicule, à la dose de 0,5 mg/kg (15 rats).

Les animaux reçoivent leur traitement respectif par voie intra-péritonéale une demi-heure avant le passage dans l'openfield.

### 2.1.5. Résultats

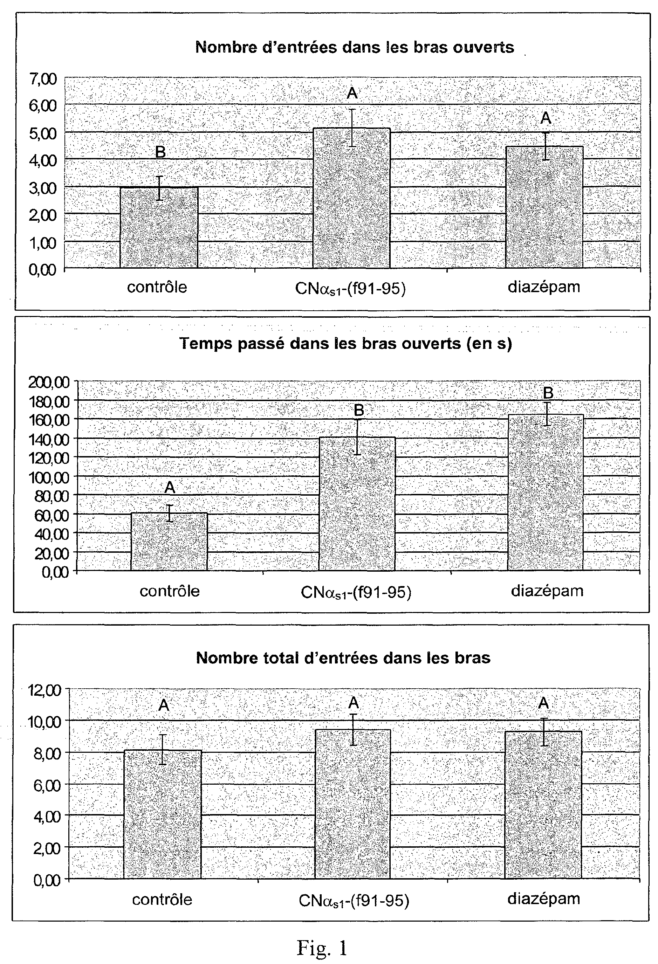

L'effet de l'injection intrapéritonéale chez le rat Wistar de 2 mL/kg d'une solution contenant 1% (v/v) de glycérol et 0,2% (v/v) de méthylcellulose (n=15) ou de 1 mg/kg de diazépam (n=15) ou de 0,5 mg/kg du pentapeptide CNαₛ₁-f(91-95) (n=15) sur (i) le nombre total d'entrées dans les différents bras, (ii) le nombre d'entrées dans les bras ouverts, (iii) le temps passé dans les bras ouverts a été étudié par analyse de variance non paramétrique (Kruskall-Wallis) et les comparaisons de moyennes ont été réalisées par le test de Mann-Whitney (A, B moyennes pour chaque traitement significativement différentes *p*<0,05, figure 1). L'activité exploratoire de l'animal, reflétée par le nombre d'entrée dans l'ensemble des bras, n'est pas significativement différente selon le traitement. Le nombre d'entrées dans les bras ouverts et le temps passé dans les bras ouverts des animaux traités avec le véhicule sont significativement inférieurs à ceux des animaux traités par le diazépam et à celle des animaux traités par le fragment 91-95 de la caséine αₛ₁. Aucune différence significative n'est constatée entre le diazépam et le fragment 91-95 de la caséine αₛ₁. Le fragment 91-95 de la caséine αₛ₁ présente, dans le test du labyrinthe en croix surélevé chez le rat Wistar, une action similaire à celle exercée par le diazépam c'est-à-dire une augmentation de l'exploration des zones anxiogènes

### 2.2. Boîte claire/obscure

### 2.2.1. Principe

Les tests clair/obscur sont basé sur l'aversion innée des rongeurs pour les environnements fortement éclairés et sur leur comportement exploratoire spontané en réponse à des facteurs de stress légers comme un nouvel environnement et la lumière. Le dispositif de la boîte claire/obscure permet d'étudier le comportement exploratoire du rongeur vis-à-vis d'un compartiment non familier aversif (fortement éclairé en lumière blanche) après habituation dans un compartiment non aversif (sombre) qui devient alors familier [BOURIN et HASCOËT, 2003 (17)]. Les anxiolytiques classiques peuvent être détectés en utilisant ce dispositif.

### 2.2.2. Appareillage et protocole

La boîte claire/obscure mesure 65 x 49 x 35 cm (h x L x 1) et est séparée en deux compartiments identiques par une plaque percée de trois portes de 8 x 8 cm. Le sol est recouvert de sciure. Chaque rat est placé pendant 24 h dans le compartiment arrière de la boîte, les portes de communication avec le compartiment avant étant inaccessibles. Le compartiment arrière devient ainsi le compartiment familier. L'animal est nourri et hydraté *ad libittum.* Le jour du test, le rat, après traitement, est replacé dans le compartiment familier et les portes communicant avec le compartiment avant (non familier) sont rendues accessibles afin que l'animal puisse explorer librement l'environnement. Le compartiment non familier est rendu aversif par un éclairage en lumière blanche d'une intensité de 1500 lux. Les animaux sont observés pendant 10 min et le temps passé dans chacun des compartiments est mesuré.

### 2.2.3. Traitements

L'étude a portée sur 36 rats répartis en trois groupes :
- un groupe témoin (12 rats) ayant reçu 2 mL/kg d'une solution contenant 1% (v/v) de glycérol et 0,2% (v/v) de méthylcellulose (véhicule),
- un groupe traité avec du diazépam (Valium^{®}, Roche, Neuilly-sur-Seine, France) à la dose de 1 mg/kg mis en suspension dans le véhicule (12 rats) servant de contrôle positif,
- un groupe traité avec le fragment 91-95 de la caséine αₛ₁, préalablement dissous dans le véhicule, à la dose de 0,5 mg/kg (12 rats).

Les animaux reçoivent leur traitement respectif par voie intra-péritonéale une demi-heure avant l'ouverture de la communication entre le compartiment familier et le compartiment non-familier.

### 2.2.4. Résultats

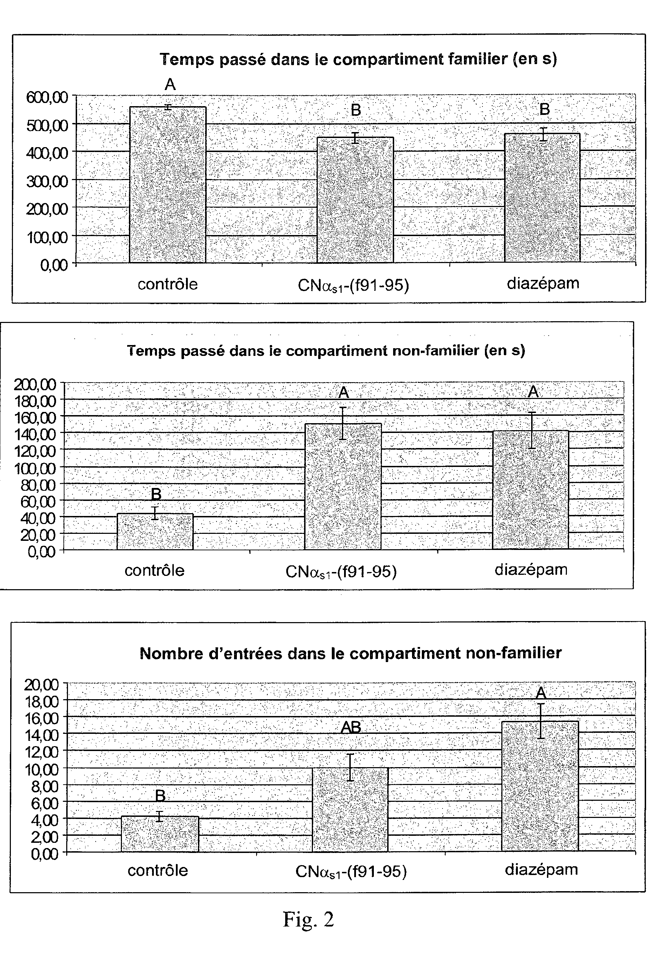

L'effet de l'injection intrapéritonéale chez le rat Wistar de 2 mL/kg d'une solution contenant 1% (v/v) de glycérol et 0,2% (v/v) de méthylcellulose (n=12) ou de 1 mg/kg de diazépam (n=12) ou de 0,5 mg/kg du pentapeptide CNαₛ₁-f(91-95) (n=12) sur (i) le nombre d'entrées dans le compartiment non-familier, (ii) le temps passé dans le compartiment familier et (iii) le temps passé dans le compartiment non-familier a été étudié par analyse de variance non paramétrique (Kruskall-Wallis) et les comparaisons de moyennes ont été réalisées par le test de Mann-Whitney (A, B moyennes pour chaque traitement significativement différentes *p*<0,05, figure 2). Le temps passé dans le compartiment non-familier, aversif, des animaux traités avec le véhicule est significativement inférieur à celui des animaux traités par le diazépam et à celui des animaux traités par le fragment 91-95 de la caséine α_{s1.} Parallèlement, le temps passé dans le compartiment familier des animaux traités avec le véhicule augmente significativement. Aucune différence significative n'est constatée entre le diazépam et le fragment 91-95 de la caséine αₛ₁. Le fragment 91-95 de la caséine αₛ₁ montre une action, de type anxiolytique, similaire à celle du diazépam dans le test de la boîte claire/obscure chez le rat Wistar.

### RÉFÉRENCES BIBLIOGRAPHIQUES

(1) MERCIER, J.C., GROSCLAUDE, F., and RIBADEAU-DUMAS, B., 1971, Structure primaire de la caséine αs1 bovine. Séquence complète. Eur. J. Biochem., 23, 41-51.
(2) NAGAO, M., MAKI, M., SASAKI, R., and CHIBA, H., 1984, Isolation and sequence analysis of bovine αs1-casein cDNA clone. Agaric. Biol. Chem., 48, 1663-1667.
(3) ZIOUDROU, C., STREATY, R.A., and KLEE, W.A., 1979, Opioid peptides derived from food proteins: the exorphins. J. Biol. Chem., 254, 2446-2449.
(4) LOUKAS, S., VAROUCHA, D., ZIOUDROU, C., STREATY, R.A., and KLEE, W.A., 1983, Opiod activities and structure of α-casein-derived exorphins. Biochemistry, 22, 4567-4573.
(5) MARUYAMA, S., and SUZUKI, H., 1982, A peptide inhibitor of angiotensin I converting enzyme in the tryptic hydrolysate of casein. Agric. Biol. Chem., 46, 1393-1394.
(6) MARUYAMA, S., MITACHI, H., AWAYA, J., KURONO, M., TOMIZUKA, N., and SUZUKI, H., 1987, Angiotensin I-converting enzyme inhibitory activity of the C-terminal hexapeptide of αs1-casein. Agric. Biol. Chem., 51, 2557-2561.
(7) MICLO, L., PERRIN, E., DRIOU, A., BOUDIER, J.-F., IUNG, C., and LINDEN G., 1995, Utilisation d'un décapeptide à activité de type benzodiazépine pour la préparation de médicaments et de compléments alimentaires. Brevet européen n°EP 0714910A1.
(8) MICLO, L., PERRIN, E., DRIOU, A., PAPADOPOULOS, V., BOUJRAD, N., VANDERESSE, R., BOUDER, J.-F., DESOR, D., LINDEN, G., and GAILLARD, J.-L., 2001, Characterization of α-casozepine, a tryptic peptide from bovine αs1-casein with benzodiazepine-like activity. FASEB J. (June 8, 2001) 10.1096/fj.00-0685fje.
(9) LECOUVEY, M., FROCHOT, C., MICLO, L., ORLEWSKI, P., DRIOU, A., LINDEN, A., GAILLARD, J.-L., MARRAUD, M., CUNG, M.-T. and VANDERESSE R., 1997, Two dimentional 1H-NMR and CD structural analysis in a micellar medium of a bovin αs1-casein fragment having benzodiazepine-like properties. Eur. J. Biochem., 248, 872-878.
(10) FROCHOT, C. 1998, Étude d'un décapeptide à activité de type benzodiazépine issu d'une protéine du lait bovin. Thèse de l'Institut Polytechnique de Lorraine (INPL).
(11) NITSCHMANN, H.S., and LEHMANN, W., 1947, Zum Problem der Labwirkung auf Casein, Helv. Chim. Acta, 130, 804.
(12) THOMSON, A.R, 1984, Recent developments in protein recovery and purification. J. Chem. Tech. Biotechnol., 34B, 190-198.
(13) MAUBOIS, J.L, 1984. Separation, extraction and fractionation of milk protein components. Lait, 64, 485-495.
(14) SANOGO, T., PÂQUET, D., AUBERT, F., and LINDEN, G., 1989, Purification of αs1-casein by Fast Protein Liquid Chromatography. J. Dairy Sci., 72, 2242-2246.
(15) MERRIFIELD, R.B., 1963, Solid phase peptide synthesis. I. Synthesis of a tetrapeptide. J. Am. Chem. Soc., 85, 2149-2154.
(16) PELLOW, S., CHOPIN, P., FILE, S.E., and BRILEY, M., 1985, Validation ofopen : closed arm entries in an elevated plus-maze as a measure of anxiety in the rat. J. Neurosci. Methods, 14, 149-167.
(17) BOURIN, M., and HASCOËT, M., 2003, The mouse light/dark box test. Eur. J. Pharmacol., 463, 55-65.
(18) Guesdon et al., 2006, A tryptic hydrolysate from bovine milk alpha S1-casein improves sleep in rats subjected to chronic mild stress, 27:6, 1476-1482.

## Revendications

1. Utilisation d'un peptide selon la SEQ ID N° 1 et/ou d'un peptide selon la SEQ ID N° 2 pour la fabrication d'un médicament pour le traitement de l'anxiété, des troubles du sommeil et de l'épilepsie.

2. Utilisation d'un hydrolysat de la caséine ou de la caséine αₛ₁ et/ou d'une fraction d'hydrolysat de la caséine ou de la caséine aₛ₁, contenant une quantité efficace d'un peptide selon la SEQ ID N° 1 et/ou d'un peptide selon la SEQ ID N° 2 pour la fabrication d'un médicament pour le traitement de l'anxiété, des troubles du sommeil et de l'épilepsie.

3. Composition comprenant à titre de principe actif une quantité efficace d'un peptide selon la SEQ ID N° 1 pour l'utilisation en thérapie.

4. Composition pour utilisation pour le traitement de l'anxiété, des troubles du sommeil et de l'épilepsie **caractérisée en ce qu'**elle comprend un hydrolysat ou une fraction d'un hydrolysat de la caséine ou de la caséine αₛ₁ contenant une quantité efficace d'un peptide selon la SEQ ID N° 1 et/ou d'un peptide selon la SEQ ID N° 2.

5. Composition pour utilisation selon la revendication 3 ou 4 **caractérisée en ce qu'**elle comprend un véhicule pharmaceutique approprié.

6. Composition pour utilisation selon la revendication 3 ou 4 **caractérisée en ce qu'**elle est un produit alimentaire.

7. Produit alimentaire **caractérisé en ce qu'**il comprend une quantité efficace pour le traitement de l'anxiété, des troubles du sommeil et de l'épilepsie d'au moins un peptide selon la SEQ ID N° 1.

8. Produit alimentaire selon la revendication 7 **caractérisé en ce qu'**il comprend un hydrolysat ou une fraction d'un hydrolysat de la caséine ou de la caséine αₛ₁, contenant une quantité efficace d'un peptide selon la SEQ ID N° 1.

9. Procédé de préparation d'un hydrolysat de la caséine ou de la caséine αₛ₁, contenant une quantité efficace d'un peptide selon la SEQ ID N° 1, comprenant :
- une étape d'hydrolyse enzymatique à l'aide de la trypsine et ensuite de la pepsine ou d'un mélange d'enzymes pancréatiques, et
- une étape d'isolement ou de concentration par chromatographie liquide haute performance en phase inverse, par chromatographie liquide haute performance d'échange d'anions, par chromatographie de gel filtration de seuil 1000 Da ou par une technique de séparation sur membrane.

10. Polynucléotide isolé **caractérisé en ce qu'**il code pour un peptide selon la SEQ ID N° 1.

11. Vecteur d'expression **caractérisé en ce qu'**il comprend un polynucléotide selon la revendication 10.

12. Organisme hôte, à l'exception de l'homme, **caractérisé en ce qu'**il est transformé avec un polynucléotide codant pour un peptide selon la SEQ ID N° 1, et/ou avec un vecteur d'expression comprenant un polynucléotide codant pour un peptide selon la SEQ ID N° 1.

13. Organisme hôte transformé, à l'exclusion de l'homme, **caractérisé en ce qu'**il exprime un peptide selon la SEQ ID N° 1.

## Patentansprüche

1. Verwendung eines Peptids nach SEQ ID Nr. 1 und/oder eines Peptids nach SEQ ID Nr. 2 zur Herstellung eines Arzneimittels zur Behandlung von Angst, Schlafstörungen und Epilepsie.

2. Verwendung eines Hydrolysats von Casein oder Casein αₛ₁ und/oder einer Fraktion eines Hydrolysats von Casein oder Casein αₛ₁, das bzw. die eine wirksame Menge eines Peptids nach SEQ ID Nr. 1 und/oder eines Peptids nach SEQ ID Nr. 2 enthält, zur Herstellung eines Arzneimittels zur Behandlung von Angst, Schlafstörungen und Epilepsie.

3. Zusammensetzung, die eine wirksame Menge eines Peptids nach SEQ ID Nr. 1 als Wirkstoff umfasst, zur Verwendung in der Therapie,

4. Zusammensetzung zur Verwendung zur Behandlung von Angst, Schlafstörungen und Epilepsie, **dadurch gekennzeichnet, dass** sie ein Hydrolysat oder eine Fraktion eines Hydrolysats von Casein oder Casein aₛ₂, das bzw. die eine wirksame Menge eines Peptids nach SEQ ID Nr. 1 und/oder eines Peptids nach SEQ ID Nr. 2 enthält, umfasst.

5. Zusammensetzung zur Verwendung nach Anspruch 3 oder 4, **dadurch gekennzeichnet, dass** sie ein geeignetes pharmazeutisches Vehikel umfasst.

6. Zusammensetzung zur Verwendung nach Anspruch 3 oder 4, **dadurch gekennzeichnet, dass** sie ein Nahrungsmittel ist.

7. Nahrungsmittel, **dadurch gekennzeichnet, dass** es eine zur Behandlung von Angst, Schlafstörungen und Epilepsie wirksame Menge mindestens eines Peptids nach SEQ ID Nr. 1 umfasst.

8. Nahrungsmittel nach Anspruch 7, **dadurch gekennzeichnet, dass** es ein Hydrolysat oder eine Fraktion eines Hydrolysats von Casein oder Casein αₛ₁, das bzw. die eine wirksame Menge eines Peptids nach SEQ ID Nr. 1 enthält, umfasst.

9. Verfahren zur Herstellung eines Hydrolysats von Casein oder Casein αₛ₁, das eine wirksame Menge eines Peptids nach SEQ ID Nr. 1 enthält, wobei das Verfahren Folgendes umfasst:
- einen enzymatischen Hydrolyseschritt mithilfe von Trypsin und anschließend Pepsin oder einer Mischung von Bauchspeicheldrüsenenzymen und
- einen Isolierungs- oder Konzentrationsschritt durch Umkehrphasen-Hochleistungsflüssigkeitschromatographie, durch Anionenaustausch-Hochleistungsflüssigkeitschromatographie, durch Gelfiltrationschromatographie mit einem Schwellwert von 1000 Da oder durch eine Membrantrenntechnik.

10. Isoliertes Polynukleotid, **dadurch gekennzeichnet, dass** es für ein Peptid nach SEQ ID Nr. 1 kodiert.

11. Expressionsvektor, **dadurch gekennzeichnet, dass** er ein Polynukleotid nach Anspruch 10 umfasst.

12. Wirtsorganismus, mit Ausnahme eines Menschen, **dadurch gekennzeichnet, dass** er mit einem Polynukleotid, das für ein Peptid nach SEQ ID Nr. 1 kodiert, und/oder mit einem Expressionsvektor, der ein Polynukleotid umfasst, das für ein Peptid nach SEQ ID Nr. 1 kodiert, transformiert wird.

13. Transformierter Wirtsorganismus, mit Ausnahme eines Menschen, **dadurch gekennzeichnet, dass** er ein Peptid nach SEQ ID Nr. 1 exprimiert.

## Claims

1. Use of a peptide according to SEQ ID No. 1 and/or a peptide according to SEQ ID No. 2 for manufacturing a medicinal product for treating anxiety, sleep disorders and epilepsy.

2. Use of a hydrolysate of casein or αₛ₁ casein and/or a fraction of hydrolysate of casein or αₛ₁ casein, containing an effective quantity of a peptide according to SEQ ID No. 1 and/or a peptide according to SEQ ID No. 2 for manufacturing a medicinal product for treating anxiety, sleep disorders and epilepsy.

3. Composition comprising as an active substance an effective quantity of a peptide according to SEQ ID No. 1 for use in therapy.

4. Composition for use for treating anxiety, sleep disorders and epilepsy **characterised in that** it comprises a hydrolysate or a fraction of hydrolysate of casein or αₛ₁ casein, containing an effective quantity of a peptide according to SEQ ID No. 1 and/or a peptide according to SEQ ID No. 2.

5. Composition for use according to claim 3 or 4 **characterised in that** it comprises a suitable pharmaceutical vehicle.

6. Composition for use according to claim 3 or 4 **characterised in that** it is a food product.

7. Food product **characterised in that** it comprises an effective quantity for treating anxiety, sleep disorders and epilepsy of at least one peptide according to SEQ ID No. 1.

8. Food product according to claim 7 **characterised in that** it comprises a hydrolysate or a fraction of hydrolysate of casein or αₛ₁ casein, containing an effective quantity of a peptide according to SEQ ID No. 1,

9. Method for preparing a hydrolysate of casein or αₛ₁ casein, containing an effective quantity of a peptide according to SEQ ID No. 1, comprising:
- an enzymatic hydrolysis step using trypsin followed by pepsin or a mixture of pancreatic enzymes, and
- an isolation and concentration step by means of reverse phase high performance liquid chromatography, anion exchange high performance liquid chromatography, gel filtration chromatography having a 1000 Da threshold or by means of a membrane separation technique.

10. Isolated polynucleotide **characterised in that** it codes for a peptide according to SEQ ID No. 1.

11. Expression vector **characterised in that** it comprises a polynucleotide according to claim 10.

12. Host organism, excluding humans, **characterised in that** it is transformed with a polynucleotide coding for a peptide according to SEQ ID No. 1, and/or with an expression vector comprising a polynucleotide coding for a peptide according to SEQ ID No. 1.

13. Transformed host organism, excluding humans, **characterised in that** it expresses a peptide according to SEQ ID No. 1.
